# EUROPEAN PATENT APPLICATION

(11) **EP 1 419 750 A1**
(43) Date of publication of application: **19.05.2004**
(21) Application number: 01961411.4
(22) Date of filing: 13.08.2001
(51) Int. Cl.: A61F 5/14

(54) **IMPROVED METHOD OF PRODUCING INSOLES THAT ARE USED TO CORRECT FOOT DEFORMITIES AND THE PRODUCT THUS OBTAINED**

(71) Applicant: Villamayor Coto, José Antonio, Mexico, D.F. 06000 (MX); Mata Diego, Luis, 09004 Burgos (ES)
(72) Inventor: Villamayor Coto, José Antonio, Mexico, D.F. 06000 (MX); Mata Diego, Luis, 09004 Burgos (ES)
(74) Representative: Manzano Cantos, Gregorio
(86) International application number: PCT/MX2001/000063
(87) International publication number: WO 2003/015670

(57) **Abstract**

The invention relates to and improved method of producing insoles that are used to correct foot deformities and the product thus obtained. The inventive method comprises the following 5 steps, the first of which provides a summary of the invention, namely; (i) producing an orthotic pattern using a paper insole comprising the patient's footprint; (ii) preparing the orthotic insole by obtaining the insole once again on another sheet of paper and copying said insole on a thermo-mouldable plastic resin sheet; (iii) preparing the patient's feet to receive the selected thermo-mouldable resin sheeting; (iv) fixing the thermo-mouldable resin which has been pre-softened in a kiln, placing a paper film between the sock and the softened resin, covering all the elements with a plastic bag and sealing said bag using neoprene straps with a clasp-or Velcro-type fastener; (v) and subsequently sending the moulded orthotic insole to and orthopaedic workshop in order for it to be smoothed and lined and, depending on the specific case, for the incorporation of the corresponding unweighting elements.

## Description

### TECHNICAL FIELD OF THE INVENTION

A novel method of producing the orthotics indicated in the treatment of biomechanical alterations of the lower extremities and of affections and deformities of the feet, arranged in a series of steps resulting in an orthotic insole for the foot, is presented.

This method has the feature that said insole is moulded directly on the foot of the patient with the required orthotic correction and using very low-cost materials. In this way, the resulting orthotic insole will be far superior to the known methods, both in their production time and in the materials used, which have a very low value in comparison to those used by other systems.

Furthermore, this orthotic insole has the flexibility of being able to correct the orthotic insole after its completion, or to be altered according to the results obtained in the patient.

The orthotic insole used, which is produced and moulded on the foot of the patient, consists of a shaped plastic resin, which after the moulding thereof has a sufficient resistance in order to be completed in the laboratory, where the smoothening and finishing operations are carried out to stabilise the orthotic insole.

Consequently, the field of application of the present invention subscribes to orthotic inventions for the treatment of biomechanical alterations of the lower extremities and of the affections and deformities of the feet.

### OBJECTS OF THE INVENTION

As indicated in the field of the invention, the main object of the present invention is to correct the alterations, affections and deformities of the feet, provided that this is possible by means of using insoles.

Therefore, it is an object of the present invention to have a method for producing an orthotic insole which is moulded directly on the foot of the patient, with which it is certain that the correction will be carried out, and which will correspond exclusively to the particular alteration or deformities of the patient and will in no way be a correction established in a comparative manner to specific computed patterns.

Another goal of the present invention is to have a tailored orthotic insole, which allows subsequent corrections for the perfect adjustment thereof on the patient.

Another singular feature of this invention is that pollutant dust is not produced during the production process of the orthotic insole, since the plastic resin as well as all the materials involved do not produce it.

Another object of the present invention is to have an orthotic insole provided with resilience and flexibility, with which the patient will not have any discomfort in daily use.

Another intention of the present invention is the use of harmless materials during the production process of the orthotic insole, in addition to the fact that the plastic resin used has an allergenic feature.

Finally, the proposed method is totally hygienic, since the materials do not enter into contact with the patient and the bags used are disposable, in addition to the fact that these plastic bags have a non-contaminant character.

### BACKGROUND OF THE INVENTION

The present technological development has been the result of many tests carried out beforehand, coming to propose the proposed method as well as the orthotic insole which is today made known through the present document.

As is known, the weight of a person is supported by the feet, intermittently relieving the weight from the ligaments and transferring it to the muscles as the person changes position, bipedalism being accompanied by the help of the knee and the slight flexion of the other knee.

The foot is made up of more than thirty bones which intervene in movement and also have an arch, which corresponds to the arched form of the foot depending on the bone configuration and on the effects of the ligaments thereon. Clinical experience indicates a muscular and ligament insufficiency as the most common cause of pes plano valgus (flat feet) or pes cavo varus (high-arched feet), in which the bones are stretched and eventually change shape.

For the correction of foot alterations and deformities, it is necessary to consult a specialist who, after clinical assessment, resorts to a surgical treatment, or to correction carried out by means of an orthotic insole. This work refers to the second case, i.e., the production of an orthotic insole.

As background to the present invention, there are those orthotic insoles produced on the basis of a metal with an arch designed and covered with a leather coating, mass produced according to established patterns, such that once the features of the patient are detected, the insole is simply selected and the patient is obliged to use it.

Unfortunately, in practice, it was observed that said invention does not suitably correct foot deformations, due to the fact that each foot is substantially different by having a variant pattern, further to the fact that the patient sometimes needs some additional insoles, and finally, the use of the insole by the patient is not regular since it causes discomfort when walking, for which reason the user often opts not to use the insole thus impairing even more his or her deformity.

A second known method used consists in the use of orthopaedic shoes, carrying out the correction of shoes by means of heels which raise certain zones or sections of the shoe and force the patient to step in a different manner. This process, which is well-tried with small children, has the drawback of requiring some of those additional implements, which must be permanently used, even when the patient is in a resting position, such as sleep.

Even though good results are obtained using this method, these are observed in the long term, although sometimes the shoes and artefacts prove painful since the implements must be used all day.

A third method consists in having a model of the foot of the patient. This is achieved by making what is called a positive mould of the patient, and is achieved by two similar substantial methods: the first method uses a foam on which the patient must step to have a print of his or her foot or negative, and the second method obtains a negative mould based on plaster cast bandages.

With reference to the foams, it has been observed that these are not a suitable system, essentially because the patient does not have the necessary knowledge to know how to step on the foams correcting his or her alteration, and because the professional has few chances of carrying out the necessary corrections.

The other method, i.e. based on plaster cast bandages, requires quick-setting bandages to obtain the negative.

Once the negative is achieved in either of the two cases, the next step is to obtain the positive mould, also based on plaster cast, and to mould on the latter the thermo-mouldable material chosen for the production of the orthotic thereon.

However, the positive mould originates from the taking of a negative and has the drawback of requiring an excellent print, which if not taken accurately will have an erroneous result. This entails considering that more than one negative mould are often required, with the obvious increase in material and in cost, having to be absorbed by the patient.

Another drawback is that the positive mould is often accompanied by wrinkles and rugosities due to imperfections of the setting, for which reason the laboratory will have to firstly smoothen and adjust the positive mould when designing the orthotic insole, in order to subsequently mould a metallic or plastic material chosen for the production of the orthotic.

As will be understood, the method proves very awkward given the multitude of steps to be followed, it requires a lot of supervision in order to minimise errors, and the use of different materials affects the final cost.

The drawbacks described above can be overcome by means of using the method proposed by the present work, which has the feature of producing an orthotic insole which is moulded with the required correction for the patient, taking the positive mould directly from the foot of the patient and using very low-cost materials. In this way, the resulting orthotic insole will be far superior to the known methods, both in the production time thereof and in the materials used, since these have a very low value in comparison to those used by other systems.

### DETAILED DESCRIPTION OF THE INVENTION

The method of the present invention begins with a diagnosis step, consisting in the examination by a specialist.

Various aspects are carried out to this end, namely: a gait examination, a study of the footwear used by the patient, an examination of the bare foot and a study of the unweighted foot.

The gait examination consists in examining the movement and the gait, and goniometry (measurement of the range of active and passive joint amplitude), electromyographic and cinematographic analyses, and podobarometric systems (implemented insoles) with which the static and dynamic plantar pressure on the surface of a sensor can be analysed, are used. A podoscopic platform, consisting of a glass platform for observing the sole of the foot, and of a mirror placed under the platform, with a slight inclination for seeing the movement of the foot when the patient walks, is normally used to this end.

The examination of the footwear used by the patient is very important, as correction can take place by means of using another type of shoes.

The examination of the foot is definitive, having to take into account the general appearance and the relation thereof with the rest of the locomotor system.

Finally, the study of the unweighted foot must comprise the joint and comparative analysis of the feet, the study of the precise areas, the muscular examination, the neurological examination and the circulatory examination.

The next step is the immediate assessment and determination of the application of an orthotic insole after having obtained the diagnosis, and which is described below.

Three types of orthotic insoles to be produced are distinguished: those intended to restore the support of the affected metatarsals; those intended to correct the different alterations in the plantar arch; and those where as well as correcting the arch, it is necessary to restore the support of the affected metatarsals. However, with slight modifications, the method is totally similar.

A first step consists in elaborating a pattern on paper, which is obtained from a sheet of paper on which the patient has left a print.

To this end, firstly, lines denoting the limit up to where the insole will have to reach are drawn on the sole of the foot of the patient using an non-erasable ink pen.

Separately, a pattern of the inside of the shoe normally used by the patient is traced and cut out, placing said pattern inside the shoe. Then, the paper pattern is dipped in alcohol and the patient is asked to put his or her shoes back on, being careful not to let the pattern smudge. The patient is thus asked to walk for a few minutes after this. The alcohol will pass to the foot and will print the drawing made on the sole of the foot onto the paper, in a similar way to a stencil.

A second step consists of the preparation of the orthotic insole consisting in obtaining a second pattern on another sheet of paper, copying only the part which must be corrected, in order to subsequently draw the contour of this cut-out shape onto a resin sheet of thermo-mouldable plastic. These resins have the form of sheets of a thickness varying from 2 to 3 millimetres, one side of which is smooth whereas the other side comes with a loosely woven fabric layer, this material serving as reinforcement.

Once the contour of the shape drawn on the surface of the plastic resin sheeting has been cut out, it is placed in a kiln according to the specific temperature of each material for the purpose of softening it, the temperature of which kiln ranges between 35 and 120°C during a period of 2 to 3 minutes.

When it is desired to obtain a correction of, for example, 5 millimetres, two chosen thermo-mouldable resin sheets are usually placed, the two sheets having to be placed on their smooth sides, i.e. face to face, the sides with fabric thus facing outwards. The heat of the kiln will melt the two thermo-mouldable resin sheets forming a single piece.

A third step consists in preparing the feet of the patient to receive the chosen thermo-mouldable resin sheet. To this end, a disposable plastic sock is placed, with which the sock of the patient is separated both as a measure of hygiene between the doctor and the patient and in order to perfectly isolate the foot, said element being very simple and economical.

A heat-insulating sock is immediately placed, the function of which will be to prevent any harm to the sole of the foot of the patient due to heat.

A hose connected to a vacuum pump is placed in the sock, the function of which will be to extract all of the air.

A fourth step consists in placing the thermo-mouldable resin, pre-softened in the kiln, on the heat-insulating sock, and a paper film is placed between the sock and the softened resin so that the resin does not stick to the sock.

All the elements are covered with a plastic bag, and the bag is sealed with neoprene straps with a fastener such as clasps or an adhesive Velcro film.

The vacuum pump will extract all of the air, and at the same time, the resin sheet begins to be moulded on the foot of the patient, aiming to push the resin onto the arch. The air gaps between the foot and the resin are extracted by the vacuum pump, leaving an insole tailored to the foot of the patient.

When a cooling of the moulded resin is observed, the straps are removed and the moulded insole is withdrawn. This operation takes a couple of minutes.

The cooling time depending on each type of thermo-mouldable resin used is from 1 to 3 minutes, which allows making necessary corrections in each particular case, or simply obtaining an orthotic insole in a neutral position.

In order to guarantee the insole, and if the personnel who produced it deem necessary, the moulded part can first be placed in the shoe of the patient, and the patient can be asked to walk in order to express the improvement thereof.

A fifth step consists of the smoothening and lining of the moulded orthotic insole, consisting in sending the latter to the orthopaedic workshop for the smoothening and lining thereof, wherein the corresponding unweighting elements are incorporated depending on the specific case.

The manufacture of the mouldable orthotic insole, from the dressing of the foot of the patient with a sock until the final step, does not exceed ten to fifteen minutes, which demonstrates an important saving of time as well as of substituted plaster cast material if compared with the method of obtaining a positive mould of the foot of the patient.

As well as being moulded directly on the feet of the patient, the orthotic insole proves exact, unlike the use of a positive mould.

For the obtainment of the orthotic, only disposable socks, making it a clean method, heat-insulating socks and a small vacuum pump are required. In the final finishing, usual tools and machinery will be used for the production of the orthotics.

### BEST METHOD FOR CARRYING OUT THE INVENTION

As an example, the case of an adult who has suffered a flattening of the cavity or arch of the foot as a result of fatigue due to excess weight will be considered.

After having assessed the possibility of correcting the deficiency thereof by means of an insole, the next step is to mark the sole of the foot with a non-erasable ink pen.

The steps described by the description are followed until obtaining an orthotic insole.

The orthotic insole will be finished with a lining and an extension for the unweighting element of the toes of the feet.

It must be considered that for the case of a woman who uses very high heels, only one mouldable resin sheet can be used, unlike with for example a person having a fallen arch due to excess weight.

Having described the invention, the content of the following claims is considered as a novelty and is therefore claimed in my property:

## Claims

1. An improved method of producing insoles that are used to correct foot deformities, comprising a sequence of process steps which begin with a prior diagnosis by a specialist, consisting in the examination of the feet in order to assess and, if the patient so requires, to correct the somatic deficiency thereof by means of surgery or by means of an orthotic insole after having obtained the diagnosis, in order to proceed to the production of the orthotic insole, **characterised in that** it comprises:
A first step, consisting of the obtainment of a paper pattern of the footprint of the patient where the print of the correction which will be made has been left, for which lines denoting the limit up to where the insole will have to reach are drawn on the foot of the patient, using a non-erasable ink pen, separately proceeding to trace a pattern inside the shoe normally used by the patient, which is cut out and placed inside the shoe, continuing with the dipping of the paper pattern in alcohol and which will print the drawing made on the sole of the foot onto the paper in a similar way to a stencil, for which the patient is asked to put his or her shoes back on, being careful that the insole does not smudge;
A second step, consisting of the preparation of the orthotic insole, consisting in again obtaining the pattern of the insole on a second piece of paper, copying the part which must be corrected, drawing the contour of said cut-out figure onto a resin sheet of a thermo-mouldable plastic, and
wherein one side has thereof is smooth whereas the other is corrugated by a loosely woven fabric layer, this material serving as reinforcement, producing the base of the insole by cutting out the contour of the figure drawn on the surface of the thermo-mouldable resin sheet and placing it in a kiln according to the specific temperature of each material for the purpose of softening it, the temperature of which kiln ranges between 35 and 120°C during a period of 2 to 3 minutes;
A third step, consisting in preparing the feet of the patient to receive the chosen thermo-mouldable resin sheet, placing a disposable plastic sock, with which the sock of the patient is separated both as a measure of hygiene between the doctor and the patient and in order to perfectly isolate the foot, said element being very simple and economical, in order to subsequently place a heat-insulating sock, the function of which will be to prevent any harm to the sole of the foot of the patient due to heat, and then a hose connected to a vacuum pump in the sock, the function of which will be to extract all of the air;
A fourth step, consisting in placing a thermo-mouldable resin pre-softened in the kiln on the heat-insulating sock so that the resin does not stick to the sock, inserting a paper film between the sock and the softened resin, covering all the elements with a plastic bag and sealing the bag with neoprene straps with a fastener such as clasps or an adhesive Velcro film until observing the cooling of the resin moulded directly on the foot of the patient;
A fifth step, consisting in the smoothening and lining of the moulded orthotic insole, consisting in sending the latter to the orthopaedic workshop for the smoothening and lining thereof, where the corresponding unweighting elements are incorporated depending on the specific case, using usual tools and machinery for the production of orthotics in the final finishing.

2. An improved method of producing insoles that are used to correct foot deformities according to claim 1, **characterised in that** the resins of thermo-mouldable material have the form of sheets of different thickness for each case, and have a thickness of 2 to 3 millimetres, which creates the possibility to carry out combinations of various sheets in order to give a certain thickness.

3. An improved method of producing insoles that are used to correct foot deformities according to claim 1, **characterised in that** when it is desired to obtain a correction of 5 millimetres, two chosen thermo-mouldable resin sheets are usually placed, the two chosen thermo-mouldable resin sheets having to be placed, the two resin sheets having to be placed on their smooth sides, the sides with fabric thus facing outwards, and where the heat of the kiln will melt the two thermo-mouldable resin sheets forming a single piece.
